# EUROPEAN PATENT APPLICATION

(11) **EP 3 839 971 A1**
(43) Date of publication of application: **23.06.2021**
(21) Application number: 19218068.5
(22) Date of filing: 19.12.2019
(51) Int. Cl.: G16H 50/20

(54) **A COUGH DETECTION SYSTEM AND METHOD**

(71) Applicant: Koninklijke Philips N.V., 5656 AG Eindhoven (NL)
(72) Inventor: DEN BRINKER, Albertus Cornelis, 5656 AE Eindhoven (NL); PRIORI, Rita, 5656 AE Eindhoven (NL); NIJSEN, Tamara Mathea Elisabeth, 5656 AE Eindhoven (NL); DELLIMORE, Kiran Hamilton J., 5656 AE Eindhoven (NL)
(74) Representative: Philips Intellectual Property & Standards

(57) **Abstract**

A cough detection system and method uses a first database of physiological information relating to a user for whom cough detection is to be implemented and relating to other people likely to be in the vicinity of the user. A second database (used in real time or as a part of a system calibration) has cough data associated with the physiological information. There is a set of cough detection algorithms, each one tailored to a particular set of physiological characteristics. A cough detection algorithm is selected or constructed which is suitable for identifying coughs of the user while ignoring coughs of the other people. This selected algorithm is applied to sound collected to identify coughs of the user.

## Description

### FIELD OF THE INVENTION

This invention relates to systems and methods for detecting coughing, and in particular for detecting coughing of one target person when there may be other coughing persons present.

### BACKGROUND OF THE INVENTION

Coughing is a symptom of many respiratory diseases.

Monitoring coughing has been shown to be of clinical relevance for specific target groups like e.g. COPD patients and asthma patients, and it is therefore a key and crucial component to allow tracking of the health status of such patients over time.

Preferably, it would be possible to monitor patients in their natural environment with unobtrusive means. One convenient unobtrusive monitoring approach is by using audio sensing to determine cough utterances. These utterances can be analyzed in terms of the quantity (number of coughs) or the quality (e.g., character: dry, wet cough). The monitoring may then for example be used to assist in understanding triggers for health issues or to alert the user or a caregiver, thereby enabling early warnings and early interventions.

Unobtrusive monitoring, for example with remote (non-body) sensing implies that there will be other people's coughs which will be observed as well. Large scale application, such as in the context of advanced tele monitoring solutions, requires plug-and-play systems or systems that are easily customized.

The deployment of current unobtrusive cough monitors is hampered by various issues. A general plug-and-play cough classifier may pose an accuracy problem as soon as the number of coughs produced by non-targeted persons becomes non-negligible (relative to those of the target person). However, making a full personalized system is cumbersome and time-consuming; coughs of the targeted person have to be observed, annotated and a system has to be trained.

Adaptive systems and methods for personalization exist, but adaptation or machine learning requires an environment with not too many confounding factors during the learning phase; a situation which is not easy to guarantee.

There is therefore a need for an improved cough detection system and method.

### SUMMARY OF THE INVENTION

The invention is defined by the claims.

According to examples in accordance with an aspect of the invention, there is provided a cough detection system comprising:
a first dataset comprising first physiological information relating to a user for whom cough detection is to be implemented;
a second dataset comprising second physiological information relating to other people likely to be in the vicinity of the user;
a microphone for collecting sound from the vicinity of the user; and
a processor, wherein the processor is adapted to:
   obtain a cough detection algorithm suitable for identifying coughs based on first cough data which is associated with the first physiological information while ignoring coughs based on second cough data which is associated with the second physiological information; and
   apply said cough detection algorithm to the collected sound, thereby to identify coughs of the user.

This system enables a cough detection algorithm to be obtained (selected or constructed) which performs best at identifying the coughs of a user (wherein it is assumed in this document that the "user" of the system is the targeted individual for whom cough detection is desired), while disregarding coughs of known people who are likely to be present. Cough data is used to represent coughs of the user and coughs of the known other people so that the cough detection algorithms may be tested or constructed (either in advance or in real time). However, this makes use of cough detection algorithms tailored to particular physiological characteristics rather than tailored to actual individuals, and thereby reduces the training burden.

In a first set of examples, the processor is adapted to obtain a cough detection algorithm by constructing a cough classifier with the first cough data as a positive class and the second cough data and non-cough data as a negative class.

Thus, a classifier is built which detects coughs corresponding to the first cough data and discounts coughs corresponding to the second cough data.

In a second set of examples, the system further comprises a set of cough detection algorithms, each one tailored to a particular set of physiological characteristics, and the processor is adapted to obtain a cough detection algorithm by selecting one of the set of cough detection algorithms.

In this case, one cough detection algorithm will best match the use case, i.e. the physiological type of the user and the physiological types of the interferers. All possible combinations of persons (within the different sets of physiological characteristics) may have been previously tested, and obtaining the best cough detection algorithm involves selecting the best one, based simply on the known characteristics of the user and the interferers.

In a third set of examples, the system further comprises:
a third dataset comprising the first cough data; and
a fourth dataset comprising the second cough data,
and wherein the processor is adapted to:
   test the response of a plurality of the set of cough detection algorithms to the first and second cough data;
   select said one or more of the set of cough detection algorithms based on the response to the first and second cough data.

All three of these sets of example have in common that the cough detection algorithm which is eventually obtained is derived from cough data which represents a particular set of physiological characteristics, rather than being tailored to a specific individual person. In all cases, one set of physiological characteristics defines the user and another set or sets define the other interferers.

The plurality of the set of cough detection algorithms which are tested are those which are suitable for the physiological information of the user. There may be many such candidate cough detection algorithms, each with different selectivity having regard to the other people.

Each cough detection algorithm (or the single algorithm if one is constructed) may comprise a trained machine learning cough classifier or may make use of a cough model.

Thus, different detection algorithms are possible based on training using real cough sounds, or based on training using modelled cough sounds, or a combination. The cough sounds are those associated with the particular set of physiological parameters.

The first cough data may include at least one recording of a voluntary coughing sound of the user. This enables the testing of the cough detection algorithms using real cough sounds of the user. However, the system can operate even without any user-specific training, by simply mapping the user physiological parameters to standardized cough detection algorithms.

The second cough data may also include at least one recording of a voluntary coughing sound of one or more of the other (interfering) people. This enables the testing of the cough detection algorithms using real cough sounds of the other people who may be present.

The first cough data may also include at least one recording of a speech sound of the user. The speech sound may also be used as part of the classification. Similarly, the second cough data may include at least one recording of a speech sound of one or more of said other people.

The first and second cough data may comprise cough models which model coughing sounds based on the corresponding physiological information. In this case, the testing of the detection algorithms is based on the use of artificially generated cough sounds, selected (or generated in real time) based on the physiological parameters.

The first and second physiological information may each comprise one or more of:
age;
gender;
medical conditions;
height;
weight;
lung volume; and
body mass index.

These are parameters which influence the cough sound. The medical conditions for example relate to respiratory conditions.

The second dataset may comprise second physiological information relating to a plurality of other people likely to be in the vicinity of the user, and wherein the processor is adapted to test the response of a plurality of the set of cough detection algorithms to the second cough data for each of said plurality of other people.

Thus, the testing may involve considering each of multiple other people in turn. The selection may involve finding a minimum combined interference from the other people to the user.

The second dataset may again comprise second physiological information relating to a plurality of other people likely to be in the vicinity of the user, but the processor may instead be adapted to generate a hybrid second cough data combining cough data for each of said plurality of other people. This simplifies the testing, by creating a virtual combined person representing all of the other people.

The processor may be adapted to interpret audio relating to speech or footsteps to provide additional input to the cough detection algorithms. This additional information may assist in distinguishing the user from the other people.

The processor may be adapted to detect the presence of one or more of said other people based on portable electronic devices carried by said other people. This approach may for example use WiFi signal strengths or profiles, again to assist in distinguishing the user from the other people.

The processor is preferably adapted to provide a total cough count for the user.

The invention also provides a cough detection method comprising:
collecting sound from the vicinity of a user for whom cough detection is to be implemented;
obtaining a cough detection algorithm suitable for identifying coughs based on first cough data which is associated with first physiological information relating to the user while ignoring coughs based on second cough data which is associated with second physiological information relating to other people likely to be in the vicinity of the user; and
applying said cough detection algorithm to the collected sound, thereby to identify coughs of the user.

In a first example, obtaining a cough detection algorithm comprises:
constructing a cough classifier with the first cough data as a positive class and the second cough data and non-cough sound data as a negative class.

In a second example, obtaining a cough detection algorithm comprises:
selecting one or more from a set of cough detection algorithms, each one tailored to a particular set of physiological characteristics.

In a third example (as a development to the second example), selecting a cough detection algorithm comprises:
testing the response of a plurality of cough detection algorithms of a set of cough detection algorithms to the first and second cough data; and
selecting one or more of the set of cough detection algorithms based on the response to the first and second cough data.

This method enables a best cough detection algorithm to be used by testing the response to the known user characteristics and the characteristics of possibly interfering people.

Each cough detection algorithm may comprise a trained machine learning cough classifier or may make use of a cough model.

The first cough data may include at least one recording of a voluntary coughing sound of the user and/or the second cough data includes at least one recording of a voluntary coughing sound of one or more of said other people.

The method may be implemented in software.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 shows a cough detection system; and
Figure 2 shows a cough detection method.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a cough detection system and method in which a first database of physiological information relates to a user for whom cough detection is to be implemented and also relates to other people likely to be in the vicinity of the user. A second database (used in real time or as a part of a system calibration) has cough data associated with the physiological information. There is a set of cough detection algorithms, each one tailored to a particular set of physiological characteristics. A cough detection algorithm is selected or constructed which is suitable for identifying coughs of the user while ignoring coughs of the other people. This algorithm is applied to sound collected to identify coughs of the user.

There are three different ways to obtain the appropriate cough detection algorithm.

A first way involves obtaining a cough detection algorithm by constructing a cough classifier with first cough data (which corresponds to a user of the system for whom coughs are to be detected) as a positive class and second cough data (which corresponds to other interfering people) and other non-cough sounds as a negative class.

A second way involves obtaining a cough detection algorithm by selecting one or more from a set of cough detection algorithms, each one tailored to a particular set of physiological characteristics. Based on knowledge of the user and the interfering people, in particular their physiological characteristics, one (or a small number) of the previously constructed cough detection algorithms will be most appropriate for use. The determination of which cough detection algorithms are appropriate for different combinations of a user and interfering people may already be determined as a global system calibration based on testing the responses of the different cough detection algorithms to different combinations of users and interferers.

A third way involves selecting a cough detection algorithm by testing the response of a plurality of cough detection algorithms of a set of cough detection algorithms to the first and second cough data and selecting one or more of the set of cough detection algorithms based on the response to the first and second cough data.

The third approach makes use of greatest amount of data processing in the system, and for this reason a detailed example will be discussed for this third approach.

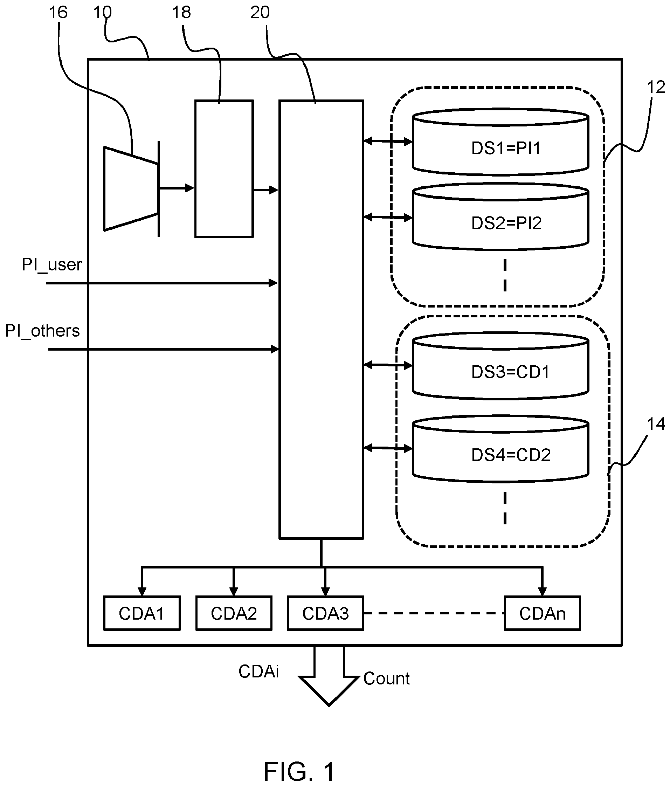

Figure 1 shows a cough detection system 10.

A first database 12 stores physiological information relating to both a user for whom cough detection is desired as well as for other uses who may be in the vicinity of the user.

The first database thus has a first dataset DS1 comprising first physiological information PI1 relating to a user for whom cough detection is to be implemented and a second dataset DS2 comprising second physiological information PI2 relating to other people likely to be in the vicinity of the user.

The physiological information for example comprises parameters such as age, gender, medical conditions, height, weight, lung volume, and body mass index (bmi). These parameters are largely dependent: the lung volume depends heavily on age and gender. This implies that in many cases even with partial information, sufficient data is available to have an operating system. The input data may also comprise (voluntary) coughs and speech utterances (or features thereof) as these provide relevant information on the vocal tract.

A second database 14 stores cough data relating to different actual people or different categories of people according to the physiological information characteristics.

The second database thus has a third dataset D3 which comprises first cough data CF1 associated with the first physiological information, i.e. associated with the same physiological person type as the user. The second database has a fourth dataset DS4 which comprises second cough data CD2 associated with the second physiological information, i.e. associated with the same physiological person type as the other people likely to be in the vicinity.

This cough data may be a database of recorded coughs of persons with known physiological parameters or artificially generated coughs based on models of coughs, dependent on these parameters. Also, more complex data may be involved such as characterization of speech utterances (e.g. vowel sounds or their characterization) or voluntary cough utterances (or features thereof).

The system has a set of cough detection algorithms CDA1 to CDAn. Each one is tailored to a particular set of physiological characteristics. Thus, a particular cough detection algorithm is designed to detect a cough for a particular physiological profile. There are typically multiple cough detection algorithms which may be suitable for particular user (i.e. a particular set of physiological parameters), and there will be some that are better at discriminating from the interfering people than others.

The system has a microphone 16 for collecting sound from the vicinity of the user. The sound signals are processed by a sound processing unit 18 to extract sound features enabling cough identification. The sound processing unit enables audio features to be extracted, such as are mel-frequency cepstral coefficients (MFCCs) and time-domain envelopes and energies.

In order to select the most appropriate cough detection algorithm, testing may be performed either as an initial calibration each time the system is used and the user and interferers are identified, or instead as a global initial calibration.

For example, all combinations of categories of user and interferer may be tested in advance of the use of the system. Once the user has been identified as well as the possible interferers, this information then enables the most appropriate cough detection algorithm to be used.

Alternatively, the testing may be based on the known user and known interferer, once these have been identified to the system.

In both cases, use is made of cough data associated with particular physiological characteristics. Thus, one or more of the set of cough detection algorithms is selected based on first cough data which is associated with the first physiological information while ignoring coughs based on second cough data which is associated with the second physiological information. The selected cough detection algorithm will also ignore non-cough sounds.

In this case, a main processor 20 tests the response of some or all of the plurality of the cough detection algorithms CD1 to CDn to the first and second cough data CD1, CD2. In this way, each cough detection algorithm can be tested to determine how well it recognizes a particular cough type, as encoded by the cough data. Typically, only a sub-set is tested, which is at least able to identify a cough for the physiological information corresponding to the user.

One (or more than one) of the set of cough detection algorithms is then chosen based on the response to the first and second cough data. The chosen cough detection algorithm is shown as CDi. This is the best cough detection algorithm for identifying coughs based on the first cough data (i.e. of the same type as the user) while ignoring coughs based on the second cough data (i.e. of the same type as the other people). All of the cough detection algorithms are of course trained or otherwise designed to ignore non-cough sounds, such a speech, footsteps, doors opening/closing slamming etc.

The chosen cough detection algorithm is then applied to the collected sound, thereby to identify coughs of the user. A count "Count" of such coughs may then be maintained and output by the system.

In addition to a count of the coughs (per unit time) a stratification may also be provided, for example relating to the nature of the cough such as wet or dry). The count can be for instance the number of coughs per hour or per day. This output can be made available to the user and/or or caregiver in the form of numerical output, a graph or a message.

This system thus enables a cough detection algorithm to be selected which performs best at identifying the coughs of a user, while disregarding coughs of known people who are likely to be present. The cough detection algorithms are tailored to particular physiological characteristics rather than tailored to actual individuals. This reduces the training burden.

In order to configure the system, the cough detection algorithms are first created, for example as initial cough classifiers based on characteristics of the user (the patient targeted for cough detection) and the dominating interfering source(s). The cough detection algorithms for example each comprise a database, a set of trained cough classifiers or one or more cough models. The most appropriate cough detector may be chosen or else calculated such that it can act as an initial classifier of the user but also having low sensitivity to the dominating interfering source.

Since coughs are produced by the respiratory system, their characteristics are also for a major part determined by the physiology of the respiratory system. Determinants of this are the size and state of the system which are in turn influenced by age, gender and, possibly, diseases.

To create the cough detection algorithms, databases of coughs of multiple persons can be collected together with the relevant physiological parameters such as age, height, bmi, gender and medical history (in particular, respiratory diseases). This may be based on machine learning. The creation of the cough detection algorithms also makes use of non-cough sounds. Examples are speech sounds, slamming doors, barking dogs, etc.

The cough detection algorithms may also make use of speech utterances or voluntary cough utterances (or an (average) feature representation thereof)). As an alternative to a database, cough models could be available for various subsets of patients. Thus, there are various ways in which cough detection algorithms can be developed for specific target groups.

The testing of the cough detection algorithms to verify that their sensitivity of to coughs of the dominant interfering source is low can be based on coughs existing in the database or using associated cough models.

The determined cough detector may be initial setting in a self-learning system such that the system is further optimized while in operation.

In many cases, a dominant interfering source (i.e. another person) is easily identified. For an unobtrusive cough monitor in the bedroom, the dominant interfering source would obviously be the bed partner. For an asthmatic child sleeping alone and having a cough monitor in the bed room, the dominant interfering sources are the parents. Thus the caregiver of the targeted person can easily obtain the required physiological parameters to input to the system so that the testing can be tailored to those individuals.

The various units shown may all be part of one system or they may be connected by wires or wirelessly. A typical set-up would have the audio sensor 16 and the sound processing unit 18 (audio feature extraction unit) in the home of the patient, and the cough detection algorithms and output unit for performing the count are with the caregiver. This set-up would allow further refinement as over time more data is collected and the initial cough detector algorithms can be refined based on the data received. The input parameters can be updated whenever deemed necessary. Furthermore, newly collected cough data can be added to the database 14 to improve the system for other patients.

A basic implementation of the system will now be outlined.

There may be only three cough detection algorithms CDA1 to CDA3. These are a cough detection algorithm for tall adults, for smaller adults and for children. A caregiver knowing the situation at home and knowing the family could be of the opinion that the voices and coughs of the family members are largely different and follow the predefined categories in the system.

The caregiver will then enter the physiological information reflecting the desired monitoring situation. For example, if there is an adult couple with a monitor in the bedroom where the largest person has to be monitored, the caregiver will input physiological information which identifies the tall adult as the targeted group (the user) and the small adult as the interfering group (the single other person). The system will then select the cough detection algorithm associated with tall adults.

For the example of pediatric asthma, the caregiver will enter physiological information of the child as the target person (user) and physiological information for the adult as the interfering source. The system will then select the cough detector optimized for children. Thus, this minimal system would already be suitable to serve a large number of cases. It is part of the caregiver's task to make sure that the monitoring system is only provided to those situations for which the available cough categories are appropriate for the actual situation being monitored.

The simple examples above assume only one interfering source. There may be more than one interfering source and none is dominant, i.e., they should be considered equally probable. In this case, physiological information of more than one interfering person is entered into the system.

In one approach, the physiological data of the more than one interfering person is combined into a single virtual person, and there is corresponding cough data generated for that virtual person. In this way, the processor may be considered to generate hybrid second cough data combining cough data for each of said interfering people. Thus, the interfering sources are represented by a virtual person providing a single model for the multiple interfering sources.

The same testing process then follows in that one or more candidate cough detection algorithms is chosen based on the characteristics of the targeted person. These one or more cough detection algorithms are tested for their sensitivity to coughs of the interfering source, which in this case is a virtual/hybrid person. Based on the operating characteristics for both the targeted person and the interfering source (virtual/hybrid person), the best cough detection algorithm is selected.

Alternatively, instead of merging the multiple interfering people into a virtual/hybrid person, each candidate cough detection algorithm for the targeted person (user) may be evaluated for a performance loss due to the presence of each interfering person in turn. Performance losses may then be added equally across all interfering persons since there is no real dominancy of the various persons. The cough detector with the least overall performance loss is selected.

In this case, the second dataset DS2 comprises second physiological information relating to a plurality of other people likely to be in the vicinity of the user. The processor tests the response of a plurality of the set of cough detection algorithms to the second cough data for each of said plurality of other people.

There are various additional options, some of which are discussed below.

A first refinement is to enable the system to exclude coughs from an individual, in sight of the cough detector, with similar characteristics to the targeted person.

Specifically, this refinement addresses the extreme situation in which two individuals have multiple similar or nearly identical physiological characteristics, such as age, gender, bmi, lung volume, lung function data, (respiratory) disease information. This results in very subtle differences in cough characteristics making it challenging for the system to distinguish the coughs of the non-targeted person from those of the targeted person.

Additional contextual audio information recorded by the system immediately before and/or after coughs are detected and may be used to improve the classification performance. For example, recorded voices (e.g., pitch) and footsteps (e.g., walking cadence) may be used as additional inputs to the cough detection algorithms.

Since voice and walking information is not always available, the unique Wi-Fi signature emitted from a portable device of the user and/or other people may be used, such as their smartphone, smart glasses or smart watch. These devices are typically worn by or carried by the targeted person at all times.

More specifically, the presence of the Wi-Fi signature and the Wi-Fi signal strength may be used to confirm that the targeted person is present within sight of the detector. For example, if the system and its microphone is in the living room and the targeted person is in the garden (outside the range of the system as determined by the weak Wi-Fi signal strength), and a cough is detected by the system, then the cough will be discounted. In this way the specificity of the classifier will be improved by reducing the number of false positives.

In a further refinement, a single cough detector may be used to monitor the coughs of two targeted persons with similar characteristics. This is accomplished by utilizing the respective unique Wi-Fi signatures of each targeted persons' portable device to help in the classification of coughs recorded by the cough detector.

This covers the situations in which:
a) targeted person 1 only is in sight of the detector
b) targeted person 2 only is in sight of the detector
c) both targeted persons are insight of the detector

In the first two cases the classification of the coughs of the two persons is made according to their non-overlapping presence in sight of the cough detector.

If coughs are detected and neither of the targeted persons is present, then the detected coughs will be discounted.

In the third case, in which both targeted persons are in sight of the cough detector, it is difficult to discriminate the coughs between the two targeted persons since they have similar characteristics. Therefore, instead of tracking individual cough counts, the total cough count of both persons may be aggregated by the system into a combined cough count. Moreover, if there is a trend indicating a significant increase in coughs over a given monitoring period (e.g., 1 week), then this will trigger clinical intervention or further investigation. For example, a nurse may pay a house-call to determine which of the targeted persons has been coughing more and then provide appropriate therapy adjustment.

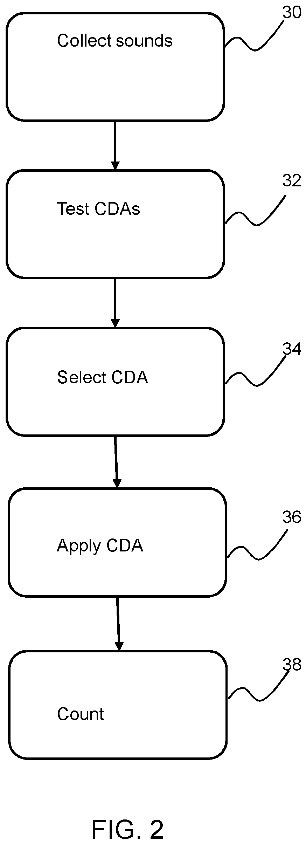

Figure 2 shows a cough detection method.

In step 30, sound is collecting from the vicinity of a user for whom cough detection is to be implemented.

In step 34 the response of a plurality of cough detection algorithms (out of a larger set of cough detection algorithms, or else the plurality may be the full set) is tested to first and second cough data.

In step 36, one or more of the set of cough detection algorithms is selected based on the response to the first and second cough data, such one or more algorithms are selected suitable for identifying coughs based on the first cough data while ignoring coughs based on the second cough data. If more than one is selected, there may be two counts and a caregiver can interpret both to judge the real cough situation.

In step 38 the one or more cough detection algorithms are applied to the collected sound, thereby to identify coughs of the user.

In step 40 a count is updated in response to each detected cough and is output to the user (or their caregiver).

The detailed example above is based on selecting a cough detection algorithm by testing the response of a plurality of cough detection algorithms to first and second cough data. As mentioned above, other approaches are possible.

The alternative approach of constructing a cough classifier with first cough data as a positive class and second cough data (and data relating to non-cough sounds) as a negative class avoids the testing step and avoids storage of a set of cough detection algorithms. It still makes use of a database of cough data and the associated physiological information, but then requires a classifier construction unit.

The alternative approach of selecting one or more from a set of previously tested cough detection algorithms again avoids the need for testing in the system in real time. The system no longer needs the database of cough data since this is used in the general calibration of the system. It still makes use of a database physiological information so that the user types and interferer types can be identified.

The invention is of particular interest for telehealth systems using home monitoring, especially for chronic respiratory diseases such as COPD and asthma.

The cough detection algorithms may be based on machine learning algorithms.

A machine-learning algorithm is any self-training algorithm that processes input data in order to produce or predict output data. Here, the input data (for each version of the cough detection algorithm, i.e. for different sets of physiological parameters) comprises coughing sounds and other sounds (such as coughs of people with other physiological parameters) and the output data comprises a cough indication (and optionally also a cough type).

Suitable machine-learning algorithms for being employed in the present invention will be apparent to the skilled person. Examples of suitable machine-learning algorithms include decision tree algorithms and artificial neural networks. Other machine-learning algorithms such as logistic regression, support vector machines or Naive Bayesian model are suitable alternatives.

The structure of an artificial neural network (or, simply, neural network) is inspired by the human brain. Neural networks are comprised of layers, each layer comprising a plurality of neurons. Each neuron comprises a mathematical operation. In particular, each neuron may comprise a different weighted combination of a single type of transformation (e.g. the same type of transformation, sigmoid etc. but with different weightings). In the process of processing input data, the mathematical operation of each neuron is performed on the input data to produce a numerical output, and the outputs of each layer in the neural network are fed into the next layer sequentially. The final layer provides the output.

Methods of training a machine-learning algorithm are well known. Typically, such methods comprise obtaining a training dataset, comprising training input data entries and corresponding training output data entries. An initialized machine-learning algorithm is applied to each input data entry to generate predicted output data entries. An error between the predicted output data entries and corresponding training output data entries is used to modify the machine-learning algorithm. This process can be repeated until the error converges, and the predicted output data entries are sufficiently similar (e.g. ±1%) to the training output data entries. This is commonly known as a supervised learning technique.

For example, where the machine-learning algorithm is formed from a neural network, (weightings of) the mathematical operation of each neuron may be modified until the error converges. Known methods of modifying a neural network include gradient descent, backpropagation algorithms and so on.

The training input data entries correspond to example various sounds. The training output data entries correspond to indication of which sounds are coughs for the particular physiological user type.

As discussed above, the system makes use of processor to perform the data processing. The skilled person would be readily capable of developing a processor for carrying out any herein described method. Thus, each step of a flow chart may represent a different action performed by a processor, and may be performed by a respective module of the processing processor.

The processor can be implemented in numerous ways, with software and/or hardware, to perform the various functions required. The processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A cough detection system (10) comprising:
a first dataset (DS1) comprising first physiological information (PI1) relating to a user for whom cough detection is to be implemented;
a second dataset (DS2) comprising second physiological information (PI2) relating to other people likely to be in the vicinity of the user;
a microphone (16) for collecting sound from the vicinity of the user; and
a processor (20), wherein the processor is adapted to:
obtain a cough detection algorithm (CDA1-CDAn) suitable for identifying coughs based on first cough data which is associated with the first physiological information while ignoring coughs based on second cough data which is associated with the second physiological information; and
apply said cough detection algorithm to the collected sound, thereby to identify coughs of the user.

2. The system of claim 1, wherein:
the processor is adapted to obtain a cough detection algorithm by constructing a cough classifier with the first cough data as a positive class and the second cough data and non-cough sound data as a negative class; or

3. The system of claim 1, further comprising a set of cough detection algorithms (CDA1-CDAn), each one tailored to a particular set of physiological characteristics, wherein the processor is adapted to obtain a cough detection algorithm by selecting one of the set of cough detection algorithms.

4. The system as claimed in claim 3, further comprising:
a third dataset (DS3) comprising the first cough data (CD1); and
a fourth dataset (DS4) comprising the second cough data (CD2),
and wherein the processor is adapted to:
test the response of a plurality of the set of cough detection algorithms to the first and second cough data;
select said one or more of the set of cough detection algorithms (CDA1-CDAn) based on the response to the first and second cough data,
and wherein each cough detection algorithm (CDA1-CDAn) comprises a trained machine learning cough classifier or makes use of a cough model.

5. The system as claimed in any one of claims 1 to 4, wherein:
the first cough data (CD1) includes at least one recording of a voluntary coughing sound of the user and/or at least one recording of a speech sound of the user; and/or
the second cough data (CD2) includes at least one recording of a voluntary coughing sound of one or more of said other people and/or at least one recording of a speech sound of one or more of said other people.

6. The system as claimed in any one of claims 1 to 5, wherein the first and second cough data (CD1, CD2) comprise cough models which model coughing sounds based on the corresponding physiological information.

7. The system as claimed in any one of claims 1 to 6, wherein the first and second physiological information (PI1, PI2) each comprises one or more of:
age;
gender;
medical conditions;
height;
weight;
lung volume; and
body mass index.

8. The system as claimed in any one of claims 1 to 7, wherein the second dataset (DS2) comprises second physiological information relating to a plurality of other people likely to be in the vicinity of the user, and wherein the processor is adapted to:
test the response of a plurality of the set of cough detection algorithms to the second cough data for each of said plurality of other people; or
generate a hybrid second cough data combining cough data for each of said plurality of other people.

9. The system as claimed in any one of claims 1 to 8, wherein the processor (20) is adapted to interpret audio relating to speech or footsteps to provide additional input to the cough detection algorithms.

10. The system as claimed in any of claims 1 to 9, wherein the processor (20) is adapted to detect the presence of one or more of said other people based on portable electronic devices carried by said other people.

11. A cough detection method comprising:
(30) collecting sound from the vicinity of a user for whom cough detection is to be implemented;
(36) obtaining a cough detection algorithm (CDA1-CDAn) suitable for identifying coughs based on first cough data which is associated with first physiological information relating to the user while ignoring coughs based on second cough data which is associated with second physiological information relating to other people likely to be in the vicinity of the user; and
(38) applying said cough detection algorithm to the collected sound, thereby to identify coughs of the user.

12. The method of claim 11, wherein obtaining a cough detection algorithm comprises:
constructing a cough classifier with the first cough data as a positive class and the second cough data and non-cough sound data as a negative class; or
selecting one or more from a set of cough detection algorithms (CDA1-CDAn), each one tailored to a particular set of physiological characteristics.

13. The method of claim 12, wherein selecting a cough detection algorithm comprises:
(34) testing the response of a plurality of cough detection algorithms of a set of cough detection algorithms to the first and second cough data; and
(36) selecting one or more of the set of cough detection algorithms based on the response to the first and second cough data,
and wherein each cough detection algorithm comprises a trained machine learning cough classifier or makes use of a cough model.

14. The method as claimed in any one of claims 11 to 13, wherein:
the first cough data (CD1) includes at least one recording of a voluntary coughing sound of the user and/or at least one recording of a speech sound of the user; and/or
the second cough data (CD2) includes at least one recording of a voluntary coughing sound of one or more of said other people and/or at least one recording of a speech sound of one or more of said other people.

15. A computer program comprising computer program code means which is adapted, when said program is run on a computer, to implement the method of any one of claims 11 to 14.
